# EUROPEAN PATENT SPECIFICATION

(11) **EP 4 228 520 B1**
(45) Date of publication and mention of the grant of the patent: **25.11.2026**
(21) Application number: 21802883.5
(22) Date of filing: 18.10.2021
(51) Int. Cl.: A61B 17/072

(54) **ANVIL BUTTRESS ATTACHMENT FOR SURGICAL STAPLING APPARATUS**
AMBOSSVERSTEIFUNGSBEFESTIGUNG FÜR CHIRURGISCHE KLAMMERVORRICHTUNG
ACCESSOIRE DE CONTREFORT D'ENCLUME D'APPAREIL D'AGRAFAGE CHIRURGICAL

(30) Priority: 19.10.2020 US 202017073545
(43) Date of publication of application: 23.08.2023
(73) Proprietor: Covidien LP, Mansfield, MA 02048 (US)
(72) Inventor: ABRAMEK, Pawel, North Haven, Connecticut 06473 (US); CAPPOLA, Kenneth M., North Haven, Connecticut 06473 (US); CHOWANIEC, Matthew J., North Haven, Connecticut 06473 (US); FERNANDES, Roanit, Hyderabad (IN)
(74) Representative: Zimmermann & Partner Patentanwälte mbB
(86) International application number: PCT/US2021/055376
(87) International publication number: WO 2022/086842

(56) References cited:
- EP-A2- 3 566 658
- EP-A2- 3 632 342
- EP-A2- 3 834 766
- US-A1- 2009 095 791

## Description

### CROSS-REFERENCE TO RELATED APPLICATIONS

### FIELD

The present application is generally related to surgical stapling apparatus, and more particularly, to anvil buttresses, anvil assemblies including anvil buttress attachment assemblies, and anvil buttress loading tools for releasably securing the anvil buttresses to the surgical stapling apparatus.

### BACKGROUND

Surgical stapling apparatus are employed by surgeons to sequentially or simultaneously apply one or more rows of fasteners, e.g., staples or two-part fasteners, to body tissue for the purpose of joining segments of body tissue together. Such apparatus generally include a pair of jaws or finger-like structures between which the body tissue to be joined is placed. When the surgical stapling apparatus is actuated, or "fired", longitudinally moving firing bars contact staple drive members in one of the jaws. The staple drive members push the surgical staples through the body tissue and into an anvil in the opposite jaw which forms the staples. If body tissue is to be removed or separated, a knife blade can be provided in the jaws of the apparatus to cut the body tissue between the lines of staples.

Surgical supports, e.g., meshes or buttress materials, may be used in combination with surgical stapling apparatus to bridge, repair, and/or reinforce tissue defects within a patient. A clinician may manually attach the buttress materials to the surgical stapling apparatus in the operating room during a surgical procedure, or utilize a surgical stapling apparatus including buttress materials pre-installed thereon, e.g., by an expensive automated attachment process. The buttress material reinforces the staple or suture line as well as covers the juncture of the tissues to reduce leakage prior to healing.

### New description page 1a (27 November 2025)

Document US 2009/095791 A1 relates to an apparatus for supplying surgical staple line reinforcement.
Document EP 3 834 766 A2 relates to anvil buttress loading for a surgical stapling apparatus.
Document EP 3 566 658 A2 relates to a universal linear surgical stapling buttress.
Document EP 3 632 342 A2 relates to universal linear buttress retention/release assemblies.

### SUMMARY

The invention is set out in the appended set of claims.

The present disclosure relates to anvil side buttress material attachment onto a loading unit of a surgical stapling apparatus. Anvil buttresses, anvil assemblies including anvil buttress attachment assemblies, and anvil buttress loading tools are designed to make anvil buttress attachment in the operating room a simple, straightforward, and cost effective procedure. For example, the anvil buttresses, anvil assemblies, and anvil buttress loading tools are configured for mechanical engagement of the anvil buttress to either of the anvil assembly or the anvil buttress loading tool, and for the transfer of the anvil buttress from the anvil buttress loading tool to the anvil assembly by sliding the anvil buttress loading tool, loaded with the anvil buttress, on to and off of the anvil assembly. Such configurations, as compared, for example, to attachment systems requiring the use of adhesives, reduce the number of components and/or assembly steps required to use the surgical stapling apparatus with an anvil buttress thereby simplifying the set-up and/or use of the surgical stapling apparatus and reducing time and/or cost to the user. As another example, the anvil buttress loading tools may be pre-loaded (e.g., by the manufacturer or prior to use in the operating room) with an anvil buttress so that a user need only load the anvil assembly with the anvil buttress via the loading tool which also simplifies the set-up and/or use of the surgical stapling apparatus and reduces the time and/or cost of the surgical procedure.

In one aspect, the present disclosure provides an anvil buttress loading system including an anvil assembly, an anvil buttress loading tool, and an anvil buttress. The anvil assembly includes an anvil plate and an anvil tip. The anvil plate has a tissue facing surface defining a plurality of staple forming pockets therein, and a proximal end portion of the tissue facing surface includes a hook assembly. The anvil buttress loading tool includes a carrier defining a cavity therein. The carrier has a support surface, with a proximal end portion of the carrier including a pair of proximal protrusions and a distal end portion of the carrier including a distal protrusion. The anvil buttress includes a body, a pair of proximal tabs extending proximally from the body, and a distal tab extending distally from the body. The body of the anvil buttress is positionable on the support surface of the anvil buttress loading tool with the pair of proximal tabs engaged with the pair of proximal protrusions and the distal tab engaged with the distal protrusion to retain the anvil buttress on the anvil buttress loading unit. The body of the anvil buttress is positionable on the tissue facing surface of the anvil assembly with a proximal end portion of the body engaged with the hook assembly and a distal end portion engaged with the anvil tip to retain the anvil buttress on the anvil assembly. The anvil buttress is transferrable from the anvil buttress loading tool to the anvil assembly.

The hook assembly of the anvil assembly may include a pair of hooks disposed on opposed sides of a central longitudinal slot defined through the tissue facing surface. Each hook may include an arm having a first end anchored to the tissue facing surface and a second end including a finger extending therefrom. The first end of the arm may be anchored to the tissue facing surface at a proximal end thereof and the second end of the arm may be disposed distal thereto. A terminal end of the finger may be biased against the tissue facing surface of the anvil assembly and movable relative to the tissue facing surface.

The anvil buttress may have a proximal window defined in the proximal end portion of the body. The proximal window may be configured to receive the fingers of the pair of hooks therethrough. The anvil buttress may have a distal window defined through a distal end portion of the anvil buttress. The distal window may be configured to receive the anvil tip therethrough.

The carrier of the anvil buttress loading tool includes longitudinal rails extending from opposed sides of the support surface. The anvil assembly is positionable between the longitudinal rails with the tissue facing surface adjacent to the support surface. The carrier of the anvil buttress loading tool may include a transverse rail extending across distal ends of the longitudinal rails and over the support surface defining a distal opening into the cavity.

In another aspect, the disclosure provides an anvil buttress loading assembly including an anvil buttress loading tool and an anvil buttress. The anvil buttress loading tool includes a carrier defining a cavity therein. The carrier has a support surface, with a proximal end portion of the carrier including a pair of proximal protrusions and a distal end portion of the carrier including a distal protrusion. The anvil buttress includes a body, a pair of proximal tabs extending proximally from the body, and a distal tab extending distally from the body. The body of the anvil buttress is positioned on the support surface, the pair of proximal tabs are engaged with the pair of proximal protrusions, and the distal tab are engaged with the distal protrusion.

The carrier of the anvil buttress loading tool includes longitudinal rails extending from opposed sides of the support surface. A plurality of axially spaced and opposed tabs extend from the longitudinal rails and over the support surface. The carrier of the anvil buttress loading tool may include a transverse rail extending across distal ends of the longitudinal rails and over the support surface defining a distal opening into the cavity. The distal protrusion may extend from the transverse rail. The anvil buttress may include a distal window defined through a distal end portion of the body, with the distal window aligned with the distal opening of the carrier.

In an aspect not explicitly claimed but considered useful for understanding the invention, the disclosure provides a loading unit including a staple cartridge assembly, an anvil assembly, and an anvil buttress. The anvil assembly includes an anvil plate and an anvil tip. The anvil plate has a tissue facing surface defining a plurality of staple forming pockets therein, with a proximal end portion of the tissue facing surface including a hook assembly. The anvil buttress includes a body having a proximal end portion releasably coupled to the anvil assembly by the hook assembly.

The hook assembly may include a pair of hooks disposed on opposed sides of a central longitudinal slot defined through the tissue facing surface of the anvil assembly. Each hook may have an arm having a first end anchored to the tissue facing surface and a second end including a finger extending therefrom. The first end of the arm may be anchored to the tissue facing surface at a proximal end thereof and the second end of the arm may be disposed distal thereto. A terminal end of the finger may be biased against the tissue facing surface of the anvil assembly and movable relative to the tissue facing surface.

The anvil buttress may have a proximal window defined in the proximal end portion of the body, with the fingers of the pair of hooks extending through the proximal window and positioned against the tissue facing surface. The anvil buttress may have a distal window defined through a distal end portion of the body, with the distal end portion of the anvil buttress coupled to the anvil assembly by engagement of the distal window around the anvil tip.

The details of one or more aspects of the disclosure are set forth in the accompanying drawings and the description below. Other aspects, as well as features, objects, and advantages of the aspects described in this disclosure will be apparent from the description and drawings, and from the claims.

### BRIEF DESCRIPTION OF DRAWINGS

FIG. 1 is a perspective view of a surgical stapling apparatus in accordance with an aspect of the present disclosure;
FIG. 2 is an exploded, perspective view of a tool assembly of the surgical stapling apparatus of FIG. 1;
FIG. 3 is a bottom, perspective view of an anvil assembly of the tool assembly of FIG. 2;
FIG. 4 is a perspective view of an anvil buttress loading tool, loaded with an anvil buttress, in accordance with an aspect of the present disclosure;
FIG. 5 is a perspective view, with parts separated, of the anvil assembly of FIG. 3 and the anvil buttress loading tool of FIG. 4;
FIG. 6 is a perspective view of the anvil assembly of FIG. 5 advanced into the anvil buttress loading tool of FIG. 5;
FIG. 7 is a cross-sectional view of the anvil assembly and the anvil buttress loading tool of FIG. 6, taken along section line 7-7 of FIG. 6;
FIG. 8 is a close-up view of a proximal portion of the anvil assembly and the anvil buttress loading tool of FIG. 7, shown with a hook assembly of the anvil assembly engaged with the anvil buttress;
FIG. 9 is a bottom, perspective view of the anvil assembly of FIG. 6 loaded with the anvil buttress;
FIG. 10 is a close-up view of a proximal portion of the anvil assembly and the anvil buttress of FIG. 9, shown with a hook assembly of the anvil assembly engaged with a proximal window defined through the anvil buttress;
FIG. 11 is a perspective view, with parts separated, of an anvil assembly and an anvil loading tool loaded with an anvil tip and an anvil buttress in accordance with an aspect of the present disclosure;
FIG. 12 is a perspective view, with parts separated, of the anvil loading tool, the anvil tip, and the anvil buttress of FIG. 11;
FIG. 13 is a cross-sectional view of the anvil buttress loading tool of FIG. 11, taken along section line 13-13 of FIG. 11;
FIG. 14 is a bottom, perspective view of a proximal end portion of the anvil assembly of FIG. 11;
FIG. 15 is a top perspective view of the anvil tip of FIG. 11;
FIG. 16 is a close-up view of a distal end portion of the anvil buttress loading tool of FIG. 11;
FIG. 17 is a cross-sectional view of the anvil buttress loading tool of FIG. 16, taken along section line 17-17 of FIG. 16;
FIG. 18 is a top, perspective view of the anvil assembly of FIG. 11 advanced into the anvil buttress loading tool of FIG. 11;
FIG. 19 is a bottom perspective view of the anvil assembly and the anvil buttress loading tool of FIG. 18;
FIG. 20 is a perspective view of the anvil assembly and the anvil buttress loading tool of FIG. 18, shown with a retainer of the anvil buttress loading tool being removed from the anvil plate;
FIG. 21 is a bottom, perspective view of the anvil assembly of FIG. 20 loaded with the anvil buttress;
FIG. 22 is a cross-sectional view of a distal end portion of the anvil assembly of FIG. 21, taken along section line 22-22 of FIG. 21;
FIG. 23 is a cross-sectional view of the surgical stapling apparatus of FIG. 1, including the anvil assembly and the anvil buttress of FIG. 21, during a firing stroke of the surgical stapling apparatus;
FIG. 24 is a top view of a distal end portion of the anvil assembly of FIG. 21;
FIG. 25 is a perspective view, with parts separated, of an anvil assembly in accordance with another aspect of the present disclosure;
FIG. 26 is a bottom, perspective view, with parts separated, of the anvil assembly of FIG. 25 and an anvil buttress;
FIG. 27 is a bottom, perspective view of the anvil buttress of FIG. 26 loaded onto the anvil assembly;
FIG. 28 is a top, perspective view of the anvil assembly of FIG. 25, with the cover plate removed, illustrating a spring assembly disposed within the anvil assembly;
FIG. 29 is a cross-sectional view of the anvil assembly of FIG. 28, shown with the spring assembly disposed in an unclamped position;
FIG. 30 is a cross-sectional view of the anvil assembly of FIG. 28, shown with the spring assembly disposed in a clamped position;
FIG. 31 is a perspective view of a distal end portion of the anvil assembly of FIG. 30;
FIG. 32 is a cross-sectional view of the distal end portion of the anvil assembly of FIG. 29, during a firing stroke of the surgical stapling apparatus of FIG. 1;
FIG. 33 is a perspective view, with parts separated, of an anvil assembly and an anvil loading tool loaded with an anvil buttress in accordance with yet another aspect of the present disclosure;
FIG. 34 is a bottom, perspective view of a proximal end portion of the anvil assembly of FIG. 33;
FIG. 35 is a perspective view, with parts separated, of the anvil buttress loading tool and the anvil buttress of FIG. 33;
FIG. 36 is a bottom, perspective view of the anvil buttress loading tool and the anvil buttress of FIG. 33;
FIG. 37 is a close-up view of a proximal end portion of the anvil buttress loading tool and the anvil buttress of FIG. 36;
FIG. 38 is a bottom, perspective view of the anvil assembly of FIG. 33 loaded with the anvil buttress;
FIGS. 39-41 are partial cross-sectional views of the anvil assembly and the anvil loading tool of FIG. 33, shown during loading of the anvil buttress onto the anvil assembly; and
FIG. 42 is a bottom, perspective view of the anvil assembly of FIG. 33 loaded with the anvil buttress.

### DETAILED DESCRIPTION

Embodiments of the present disclosure will now be described in detail with reference to the drawing figures wherein like reference numerals identify similar or identical elements. Throughout this description, the term "proximal" refers to a portion of a structure, or component thereof, that is closer to a user, and the term "distal" refers to a portion of the structure, or component thereof, that is farther from the user.

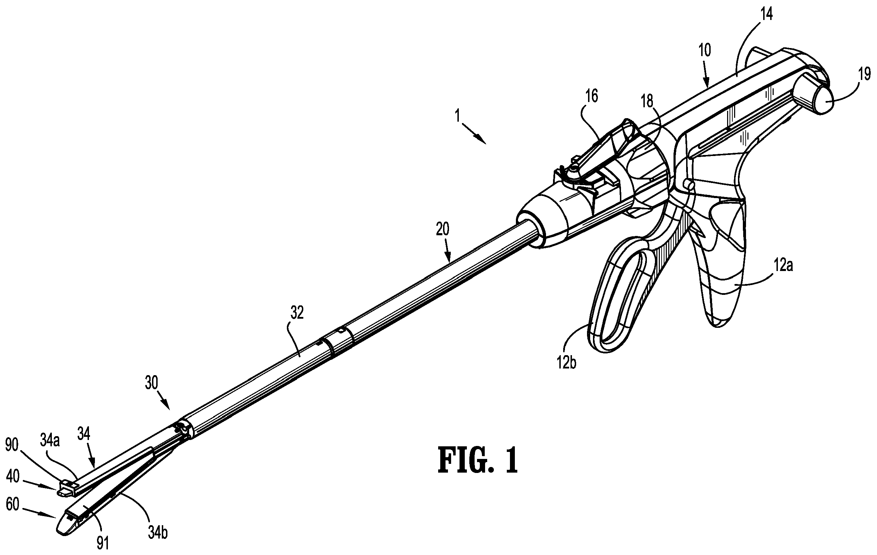

Referring now to FIG. 1, an exemplary surgical stapling apparatus or surgical stapler 1 is shown for use in stapling tissue in accordance with aspects of the present disclosure. The surgical stapling apparatus 1 generally includes a handle assembly 10, an elongate tubular body 20 extending distally from the handle assembly 10, and a loading unit 30 extending distally from the elongate tubular body 20. The loading unit 30 includes a housing portion 32 and a tool or jaw assembly 34 including first and second jaw members 34a, 34b. The first jaw member 34a and/or the second jaw members 34b is pivotable with respect to the housing portion 32 such that the tool assembly 34 is movable between an open position in which the first and second jaw members 34a, 34b are spaced apart with respect to each other, and a closed position in which the first and second jaw members 34a, 34b are substantially adjacent each other.

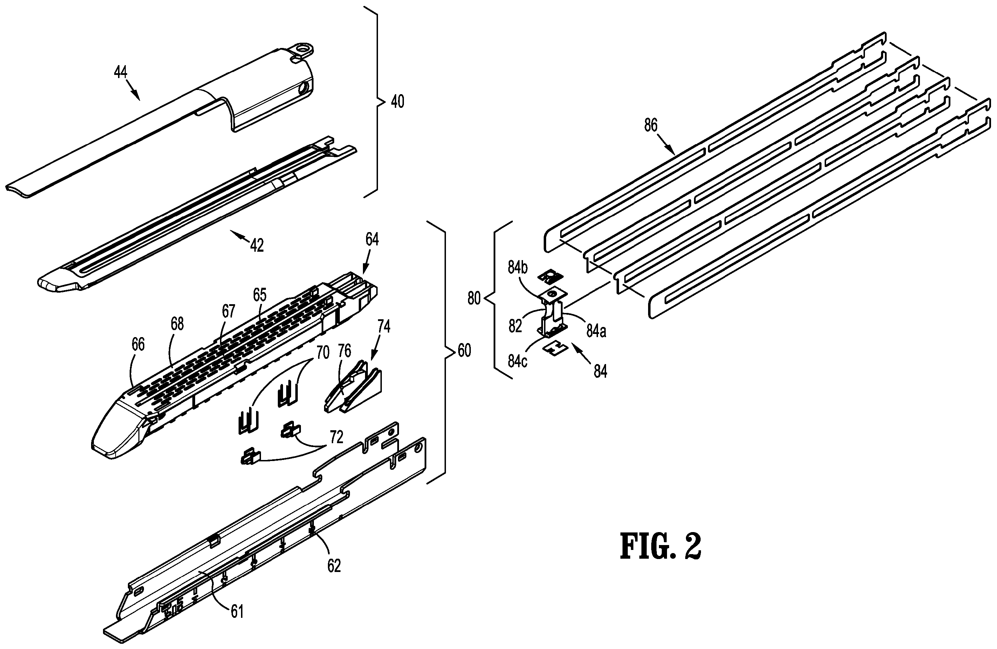

The handle assembly 10 includes a stationary handle member 12a, a movable handle member 12b, and a barrel portion 14. Actuation of the movable handle member 12b applies lines of staples 70 (FIG. 2) to tissue captured between the first and second jaw members 34a, 34b of the tool assembly 34. An articulation lever 16 is mounted on the forward end of the barrel portion 14 to facilitate articulation of the tool assembly 34. A rotatable member 18 is also mounted on the forward end of the barrel portion 14, adjacent the articulation lever 16. Rotation of the rotatable member 18 relative to the barrel portion 14 rotates the elongate tubular body 20 and the loading unit 30 relative to the handle assembly 10 so as to properly orient the tool assembly 34 relative to tissue to be stapled. A pair of knobs 19 is movably positionable along the barrel portion 14. The pair of knobs 19 is advanced distally to approximate or close the first and second jaw members 34a, 34b of the tool assembly 34 relative to each other, and retracted proximally to unapproximate or open the first and second jaw members 34a, 34b of the tool assembly 34 with respect to each other.

The loading unit 30 is a disposable loading unit ("DLU") that is releasably secured to the elongated tubular body 20 and thus, replaceable with a new loading unit 30. The loading unit 30 may be a single use loading unit ("SULU") that is used one time and then replaced to facilitate multiples uses of the surgical stapling apparatus 1 on a patient. For example, during a surgical procedure, the surgical stapling apparatus 1 can be used to staple and cut tissue, and the entire SULU is replaced after each staple and cut operation of the surgical stapling apparatus 1. The loading unit 30 may be a multi-use loading unit ("MULU") that is re-useable a predetermined number of times. For example, during a surgical procedure, the surgical stapling apparatus 1 can be used to staple and cut tissue, and a reload assembly (e.g., a staple cartridge 64 as seen in FIG. 2) of the MULU is replaced after each staple and cut operation of the surgical stapling apparatus 1 a pre-determined number of times before the entire MULU needs to be replaced. Alternatively, the loading unit 30 may be permanently affixed to the elongated tubular body 20.

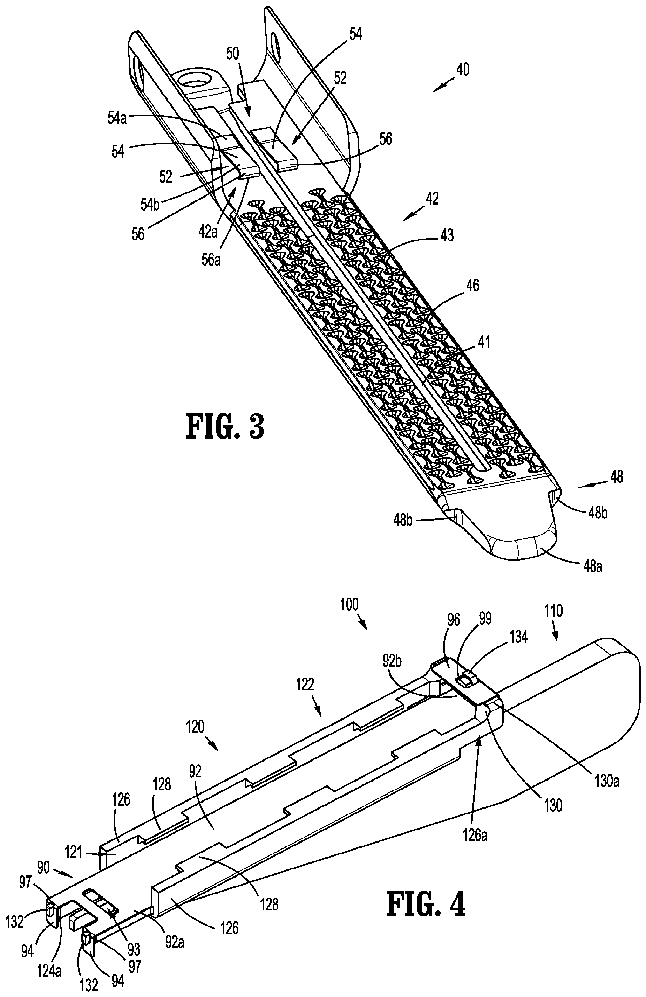

As shown in FIGS. 1 and 2, the first jaw member 34a of the tool assembly 34 includes an anvil assembly 40 and the second jaw member 34b of the tool assembly 34 includes a staple cartridge assembly 60. The anvil assembly 40 includes an anvil plate 42 and a cover plate 44 secured over the anvil plate 42. As seen in FIG. 3, in conjunction with FIG. 2, the anvil plate 42 has a central longitudinal slot 41 formed therein and a plurality of staple forming pockets or cavities 43 defined in an inward or tissue facing surface thereof 46, with an anvil tip 48 extending distal to the staple forming pockets 43. The tissue facing surface 46 is substantially planar along the length having the staple forming pockets 43 defined therein and angled outwardly or tapering towards an outer surface 45 of the anvil assembly 40 through the anvil tip 48. The anvil tip 48 has a curved leading end 48a and a pair of shoulders 48b disposed on opposed sides of the anvil tip 48. The pair of shoulders 48b are distally facing and configured to provide a stop surface for securing a distal end portion of a surgical buttress thereto. A proximal portion 42a of the anvil plate 42, which extends proximally of the staple forming pockets 43, includes a hook assembly 50 configured to secure a proximal end portion of a surgical buttress thereto.

With continued reference to FIG. 3, the hook assembly 50 includes a pair of hooks 52, with each hook 52 disposed on opposed sides of the central longitudinal slot 41. Each hook 52 includes an arm 54 having a first or proximal end portion 54a anchored or secured to the tissue facing surface 46 of the anvil plate 42 and a second or distal end portion 54b including a finger 56 extending therefrom. The finger 56 is bent or curved relative to the arm 54 such that a terminal end 56a of the finger 56 is biased against (e.g., in direct contact with) the tissue facing surface 46 of the anvil plate 42. Each hook 52 is hingedly or springedly connected to the anvil plate 52 at the proximal end portion 54a thereof such that the finger 56 is movable away from (e.g., capable of being raised off of) the tissue facing surface 46 upon application of a force thereto. In aspects, the pair of hooks 52 are leaf spring hooks, however, other cantilever hook configurations including, for example, a flexible arm and a rigid finger are contemplated.

With reference again to FIG. 2, the staple cartridge assembly 60 includes a cartridge carrier 62 defining an elongated support channel 61 configured and dimensioned to selectively receive and support a staple cartridge 64 therein. The staple cartridge 64 may be removably and/or replaceably attached to the cartridge carrier 62 by, for example, a snap-fit connection, a detent, a latch, among other types of connectors within the purview of those skilled in the art. The staple cartridge 64 includes a cartridge body 66 having an inward or tissue facing surface 68 defining staple pockets or retention slots 65 formed therein for receiving a plurality of fasteners or staples 70 and staple pushers 72. A central longitudinal slot 67 is formed in and extends along a substantial length of the cartridge body 66 to facilitate passage of a knife blade 82 of a drive assembly 80 therethrough.

The knife blade 82 is defined in a distal edge of a central wall portion 84a of an I-beam 84 that is operatively associated with the tool assembly 34. The central wall portion 84a of the I-beam 84 is slidably disposed between the anvil and staple cartridge assemblies 40, 60, with upper and lower rails 84b, 84c of the I-beam 84, respectively, supported in the anvil and staple cartridge assemblies 40, 60. The I-beam 84 is coupled to an elongated drive beam 86 which is configured to engage a drive member (not shown) of the elongated tubular body 20 (FIG. 1) of the surgical stapling apparatus 1 when the loading unit 30 is engaged therewith. The drive member imparts axial movement to the elongated drive beam 86 and thus, the I-beam 84, from the handle assembly 10. Accordingly, during operation of the surgical stapling apparatus 1, distal advancement of the I-beam 84 causes an actuation sled 74 to translate through the staple cartridge 64 and to advance cam wedges 76 of the actuation sled 74 into sequential contact with the staple pushers 72 which, in turn, cause the staple pushers 72 to translate vertically within the staple pockets 65 and urge the staples 70 from the staple pockets 65 towards the tissue facing surface 46 of the anvil plate 42 of the anvil assembly 40.

For a detailed description of the structure and function of exemplary surgical stapling apparatus, reference may be made to U.S. Patent Nos. 6,241,139, 6,330,965, and 7,819,896. It
should be appreciated that principles of the present disclosure are equally applicable to surgical stapling apparatus having other configurations such as, for example, the types described in U.S. Patent Nos. 5,964,394, 7,128,253, and 7,334,717.

Accordingly, it should be understood that a variety of surgical stapling apparatus may be utilized with aspects of the present disclosure. For example, laparoscopic or open staplers, such as, for example, GIA^{™}, Endo GIA^{™}, TA^{™}, and Endo TA^{™} staplers and/or linear and radial reloads with, for example, Tri-Staple^{™} technology, available through Medtronic (North Haven, CT) may be utilized with aspects of the present disclosure.

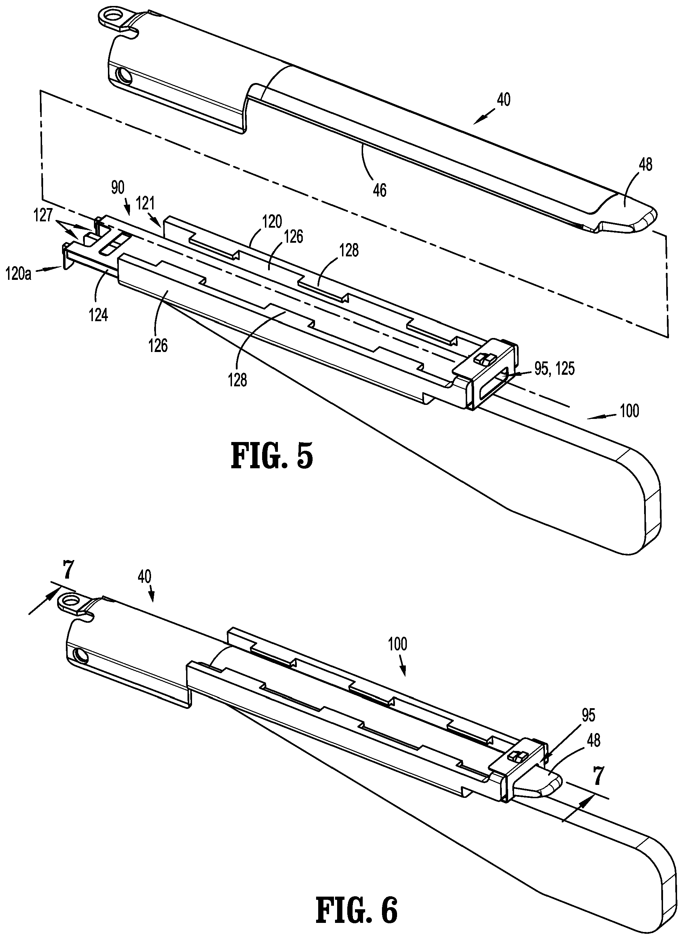

Turning now to FIG. 4, an anvil buttress 90 (also referred to herein generally as a surgical buttress) is shown loaded within an anvil buttress loading tool 100 (also referred to herein as a loading tool). The anvil buttress 90 includes a body 92 having a first or proximal window 93 defined through a proximal end portion 92a of the body 92 and a second or distal window 95 (FIG. 5) defined through a distal end portion 92b of the body 92. A pair of proximal tabs 94 extend proximally from the proximal end portion 92a of the body 92, with each tab 94 defining an opening 97 therethrough. A distal tab 96 extends distally from the distal end portion 92b of the body 92 and defines an opening 99 therethrough.

The anvil buttress 90 is fabricated from biocompatible materials which are bioabsorbable or non-absorbable, natural or synthetic materials. It should be understood that a single or combination of natural, synthetic, bioabsorbable, and/or non-bioabsorbable materials may be used to form the anvil buttress 90. In aspects, the anvil buttress 90 is formed from a single sheet of material that is cut to shape. In other aspects, the anvil buttress 90 is formed from a plurality of sheets of material, that are fabricated from the same or different materials, and/or the components (e.g., the body, the tabs, etc.) of the anvil buttress 90 are formed from the same or different materials that are attached to one another by, for example, welding, using adhesive, tying sutures, etc.

The anvil buttress 90 may be porous, non-porous, or combinations thereof Suitable porous structures include, for example, fibrous structures (e.g., knitted structures, woven structures, and non-woven structures) and/or foams (e.g., open or closed cell foams). Suitable non-porous structures include, for example, films. The anvil buttress 90 may be a single porous or non-porous layer, or include a plurality of layers including any combination of porous and non-porous layers. For example, the anvil buttress may include multiple porous and non-porous layers that are stacked in an alternating manner. In another example, the anvil buttress may be formed in a "sandwich-like" manner wherein the outer layers are porous and the inner layer(s) are non-porous, or vice versa.

Porous layer(s) in a surgical buttress may enhance the ability of the surgical buttress to absorb fluid, reduce bleeding, and/or seal a wound. Also, the porous layer(s) may allow for tissue ingrowth to fix the surgical buttress in place. Non-porous layer(s) in a surgical buttress may enhance the ability of the surgical buttress to resist tears and perforations during the manufacturing, shipping, handling, and/or stapling processes. Also, non-porous layer(s) may retard or prevent tissue ingrowth from surrounding tissues thereby acting as an adhesion barrier and preventing the formation of unwanted scar tissue.

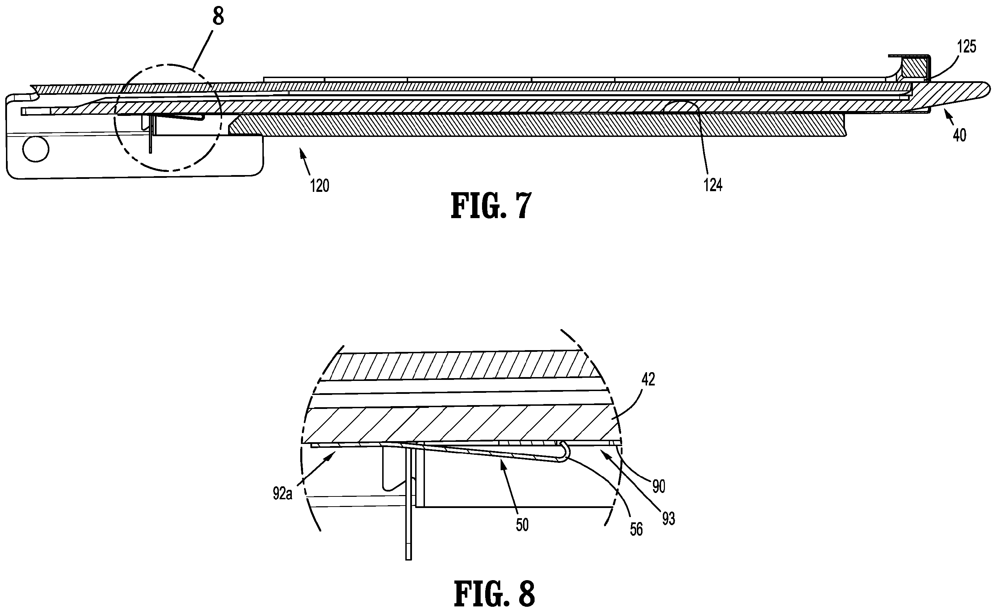

With continued reference to FIG. 4, the anvil buttress loading tool 100 includes a handle 110 and a carrier 120 secured to the handle 110. The handle 110 is configured for grasping by a user and the carrier 120 is configured to releasably retain the anvil buttress 90 therein and to receive the anvil assembly 40 (FIG. 3) for loading of the anvil buttress 90 thereon. The carrier 120 includes a housing 122 having a support surface 124 (FIG. 7), longitudinal rails 126 extending from opposed sides of the support surface 124, and a plurality of axially spaced and opposed tabs 128 extending from the longitudinal rails 126 and over the support surface 124. A cavity 121 is defined in the carrier 120 by the support surface 124, the longitudinal rails 126, and the tabs 128. A transverse rail 130 extends across distal ends 126a of the longitudinal rails 126 and over the support surface 124. The transverse rail 130 is disposed in spaced relation relative to the support surface 124 and defines a distal opening 125 (FIG. 5) into the cavity 121. A pair of proximal protrusions 132 extend proximally from a proximal end 124a of the support surface 124, and a distal protrusion 134 extends outwardly from an outer surface 130a of the transverse rail 130.

In a method of loading the loading tool 100 with the anvil buttress 90, the body 92 of the anvil buttress 90 is placed against the support surface 124 of the carrier 120. With the body 92 of the anvil buttress 90 positioned on the support surface, 124, the pair of proximal tabs 94 at the proximal end portion 92a of the body 92 is aligned or in registration with the pair of proximal protrusions 132 on the proximal end 124a of the support surface 124, and the distal tab 96 at the distal end portion 92b of the body 92 is aligned or in registration with the distal protrusion 134 of the transverse rail 130. The proximal and distal end portions 92a, 92b of the anvil buttress 90 are manipulated to engage the pair of proximal tabs 94 with the pair of proximal protrusions 132 and the distal tab 96 with the distal protrusion 134. As seen in FIG. 5, in the loaded configuration, the distal window 95 defined through the anvil buttress 90 is aligned with the distal opening 125 of the loading tool 100. It should be understood that other attachment features are contemplated for securing the tabs of the anvil buttress to the carrier of the loading tool.

As shown in FIGS. 5-8, in a method of loading the anvil assembly 40 with the anvil buttress 90, the anvil tip 48 of the anvil assembly 40 is aligned with a proximal end 120a of the carrier 120 of the loading tool 100. The anvil assembly 40 is slid distally into the cavity 121 of the carrier 120 such that the tissue facing surface 46 of the anvil assembly 40 is adjacent to or in contact with the anvil buttress 90. The anvil assembly 40 is guided through and retained within the cavity 121 of the carrier 120 by the support surface 124, the longitudinal rails 126, and the plurality of tabs 128. The anvil assembly 40 is slid distally until the anvil tip 48 extends through the distal window 95 defined through the anvil buttress 90, as seen in FIG. 6, the pair of shoulders 48b (FIG. 3) of the anvil tip 48 acting as stop surfaces to the distal movement. During this sliding movement, the hook assembly 50 slides into openings 127 (FIG. 5) defined in the proximal end 124a of the support surface 124 and captures the proximal end portion 92a of the anvil buttress 90, as seen in FIGS. 7 and 8, through placement of the fingers 56 within the proximal window 93 of the anvil buttress 90. It should be understood that additionally or alternatively, the loading tool 100 may be slid proximally relative to the anvil assembly 40.

Once the anvil assembly 40 is fully advanced into the carrier 120 of the loading tool 100, as seen in FIG. 6, the loading tool 100 may be separated from the anvil assembly 40 by sliding the loading tool 100 and/or the anvil assembly 40 away from each other. As the anvil assembly 40 and the loading tool 100 are separated from each other, the proximal and distal end portions 92a, 92b of the body 92 of the anvil buttress 90 disengage from the proximal and distal protrusions 132, 134 of the carrier 120.

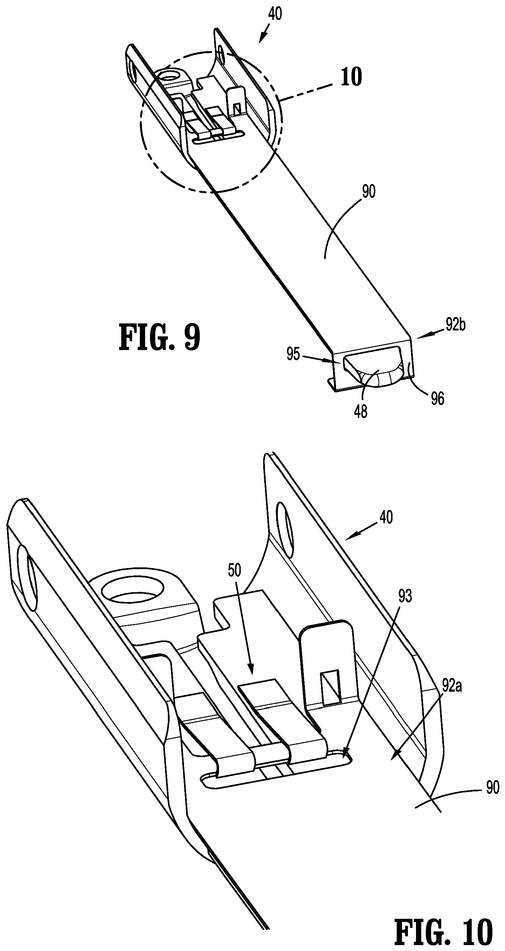

As shown in FIGS. 9 and 10, the proximal end portion 92a of the anvil buttress 90 is retained on the anvil assembly 40 by engagement of the hook assembly 50 through the proximal window 93 and the distal end portion 92b of the anvil buttress 90 is retained on the anvil assembly 40 by engagement of the anvil tip 48 with the distal tab 96 through the distal window 95. The surgical stapling apparatus 1 (FIG. 1), with the anvil assembly 40 loaded with the anvil buttress 90, is ready for use. In aspects, as seen in FIG. 1, the staple cartridge assembly 60 is pre-loaded and/or loaded with a cartridge buttress 91.

In operation, with the loading unit 30 loaded with the anvil buttress 90, as described above, the surgical stapling apparatus 1 is used in accordance with methods known by those skilled in the art. Once the anvil and staple cartridge assemblies 40, 60 are clamped onto tissue, the surgical stapling apparatus 1 is fired, thereby stapling the anvil buttress 90 to the tissue. During firing, the knife blade 82 of the I-beam 84 travels distally through the tool assembly 34 and substantially simultaneously cuts and divides the tissue and the anvil buttress 90 disposed between the rows of formed staples 70. When firing is complete and the anvil and staple cartridge assemblies 40, 60 are unclamped, the anvil buttress 90, which is now stapled to the tissue, pulls away from the anvil assembly 40, and the tool assembly 34 can be removed from the surgical site. The used staple cartridge 64 may then be removed from the tool assembly 34 and replaced with a new staple cartridge 64. A new anvil buttress 90 may be installed onto the anvil assembly 40, as needed or desired, as described above.

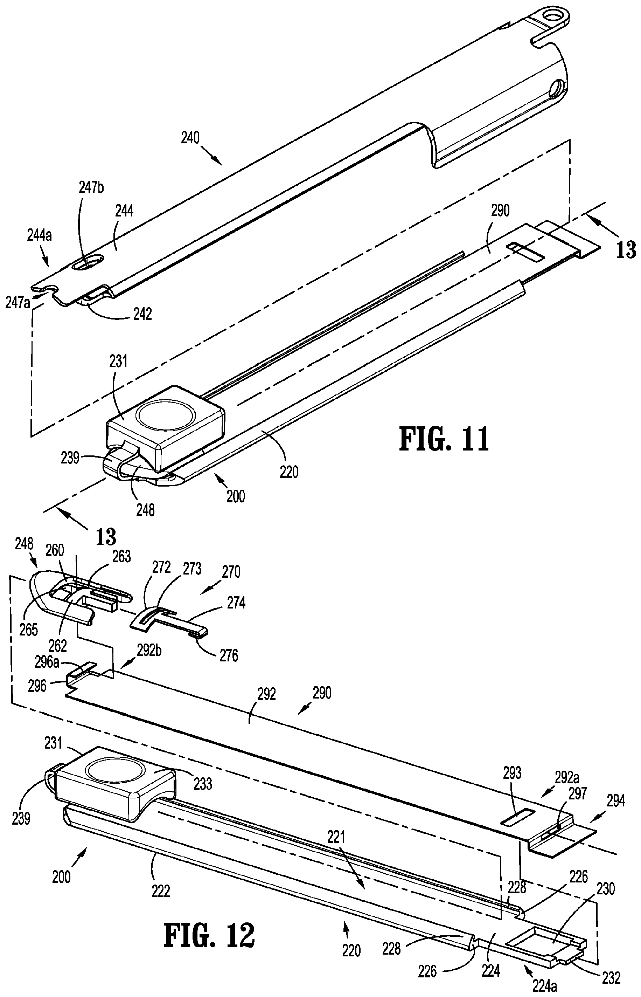

Turning now to FIG. 11, an anvil assembly 240 and an anvil buttress loading tool 200 loaded with an anvil buttress 290 and an anvil tip 248 are shown in accordance with other aspects of the present disclosure is shown. The anvil tip 248 is releasably couplable to the anvil assembly 240 and is loaded onto the anvil assembly 240 with the anvil buttress 290 coupled thereto by the loading tool 200.

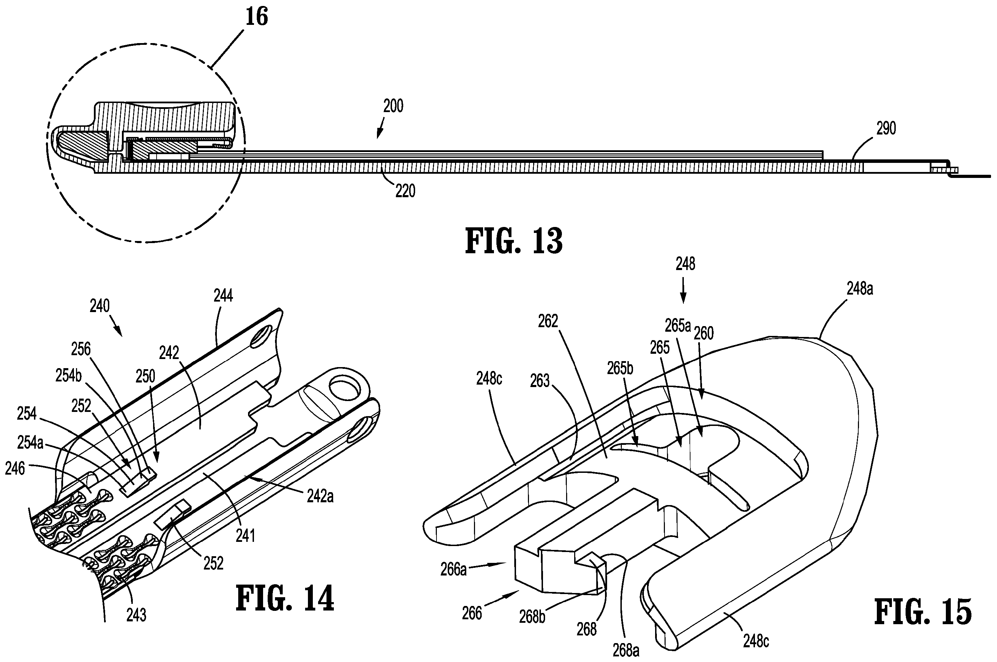

As shown in FIG. 11, in conjunction with FIGS. 14 and 15, the anvil assembly 240 includes an anvil plate 242 and a cover plate 244 secured over the anvil plate 242. An anvil tip 248 is separable from the anvil plate 242. The anvil plate 242 has a central longitudinal slot 241 formed therein and a plurality of staple forming pockets or cavities 243 defined in an inward or tissue facing surface thereof 246. A proximal end portion 242a of the anvil plate 242, which extends proximally of the staple forming pockets 243, includes a hook assembly 250 configured to secure a proximal end portion of a surgical buttress thereto.

The hook assembly 250 includes a pair of hooks 252, with each hook 252 disposed on opposed sides of the central longitudinal slot 241. Each hook 252 includes an arm 254 having a first or distal end portion 254a anchored or secured to the tissue facing surface 246 of the anvil plate 242 and a second or proximal end portion 254b including a finger 256 extending therefrom. The arm 254 extends upwardly away from the tissue facing surface 246 such that the finger 256 extends proximally and in spaced relation relative to the tissue facing surface 246 of the anvil assembly 240.

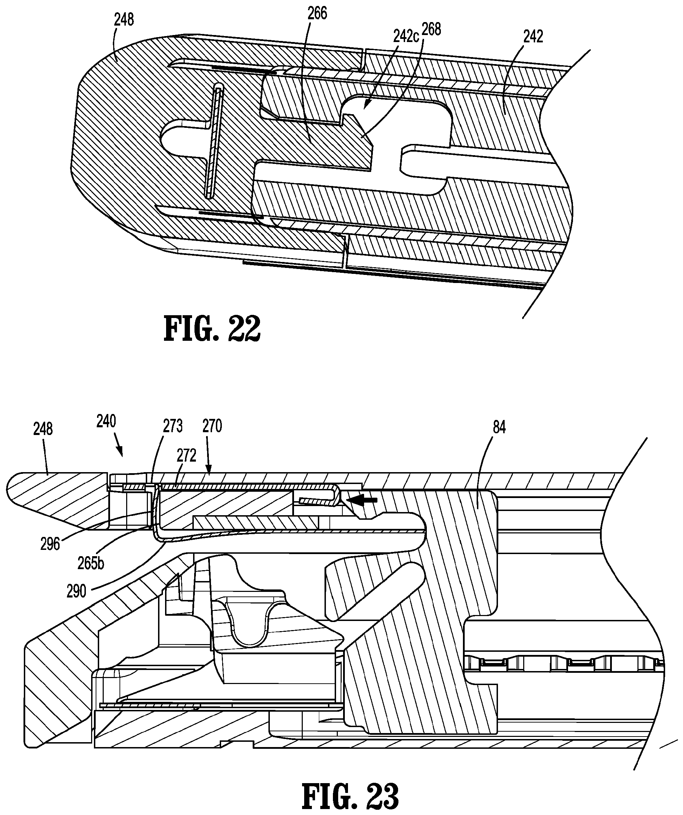

The cover plate 244 includes a notch 247a defined in a distal end 244a thereof and an opening 247b defined therethrough that is in registration with a groove 242c (FIG. 22) defined through the anvil plate 242.

The anvil tip 248 has a curved leading end 248a and a pair of legs 248c extending proximally from the curved leading end 248a. The pair of legs 248c are configured to receive the anvil plate 242 therebetween. A pocket 260 is defined in the anvil tip 248. The pocket 260 includes a curved surface 262 and longitudinal side slots 263 defined in opposed sides of pocket 260. An opening 265 extends through the curved surface 262, and includes a spherical distal region 265a and a slotted proximal region 265b.

A cantilever beam 266 extends proximally from the curved surface 262 between the pair of legs 248c. The cantilever beam 266 includes a catch 268 disposed at a proximal end 266a thereof. The catch 268 includes an engagement surface 268a to connect the anvil tip 248 to the anvil plate 242, and a cam surface 268b for deflecting the cantilever beam 266 laterally to disconnect the anvil tip 248 from the anvil plate 242.

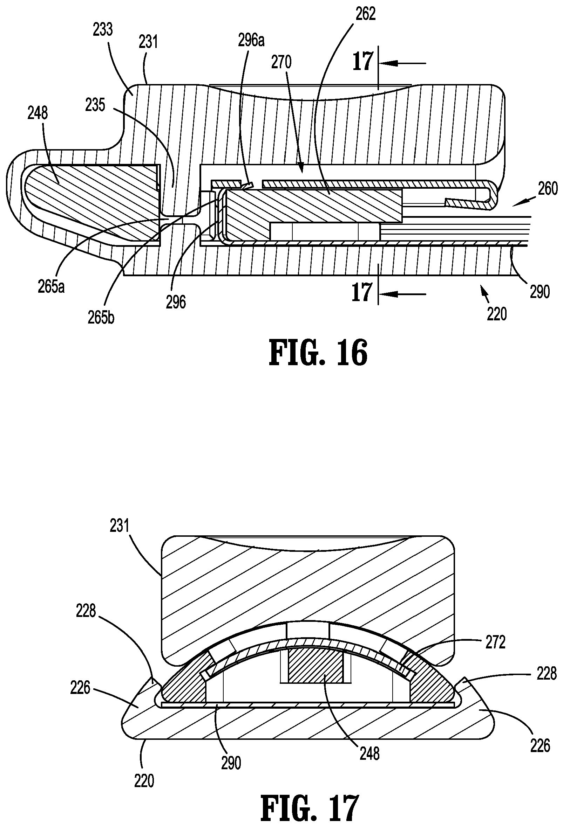

Referring now to FIG. 12, the anvil tip 248 includes a retention assembly 270 configured to secure a distal end portion 292b of the anvil buttress 290 thereto. The retention assembly 270 includes a plate 272 and an elongated bar 274 extending proximally from the plate 272. The elongated bar 274 terminates in a curved tail 276. The plate 272 is contoured (e.g., curved) for positioning within the pocket 260 defined in the anvil tip 248. The plate 272 is positionable against the curved surface 262 of the anvil tip 248 with longitudinal edges of the plate 272 received within the longitudinal side slots 263 of the pocket 260 and is slidable therein between proximal and distal positions. The plate 272 includes a slot 273 defined therethrough that is configured to align with the slotted proximal region 265b of the opening 265 extending through the curved surface 262 of the anvil tip 248 when the plate 272 is moved to the distal position. The elongated bar 274 is configured to be engaged by the I-beam 84 (FIG. 2) of the drive assembly 80 during a firing stroke to slide the retention assembly 270 from the proximal position (biased position, as seen in FIG. 16) to the distal position (as seen in FIG. 23). The curved tail 276 of the retention assembly 270 may be deformable to allow the retention assembly 270 to move between the proximal and distal positions.

As shown in FIGS. 11-13, the anvil buttress 290 includes a body 292 having a proximal end portion 292a including a proximal window 293 defined therethrough and a stepped proximal tab 294 extending therefrom. The stepped proximal tab 294 includes an opening 297 defined therethrough. The body 292 has a distal end portion 292b including a distal tab 296 extending therefrom. The distal tab 296 has a generally C-shaped configuration including a lip 296a extending substantially parallel to the body 292 in spaced relation relative thereto.

With continued reference to FIGS. 11-13, the anvil buttress loading tool 200 includes a carrier 220, a retainer 231, and a tether 239 interconnecting the carrier 220 and the retainer 231. The tether 239 is a flexible strap, cord, band, rope, cable, etc. about which the retainer 231 can move relative to the carrier 220. The retainer 231 has a base 233 and a peg 235 (FIG. 16) extending from the base 233. The carrier 220 includes a housing 222 having a support surface 224 and longitudinal rails 226 extending from opposed sides of the support surface 224, with each of the longitudinal rails 226 includes a flange 228. A cavity 221 is defined in the carrier 220 by the support surface 224 and the longitudinal rails 226. The support surface 224 includes a proximal end portion 224a extending proximally beyond the longitudinal rails 226. The proximal end portion 224a includes a window 230 defined therethrough and a proximal protrusion 232 extending proximally therefrom.

In a method of loading the loading tool 200 with the anvil buttress 290 and the anvil tip 248, the body 292 of the anvil buttress 290 is placed against the support surface 224 of the carrier 220 such that opening 297 defined through the proximal tab 294 engages the proximal protrusion 232 of the support surface 224. The anvil tip 248 is placed into a distal end portion 220b of the carrier 220 by sliding the anvil tip 248 through the cavity 221 of the carrier 220 such that longitudinal edges of the anvil tip 248 engage the flanges 228 of the longitudinal rails 226, as seen in FIG. 17. During positioning of the anvil tip 248 within the carrier 220, the distal tab 296 of the anvil buttress 290 is threaded through the slotted proximal region 265b of the opening 265 defined in the anvil plate 248 such that a terminal end 296a of the distal tab 296 rests against the curved surface 262. The retention assembly 270 is then placed into the pocket 260 of the anvil tip 248, with the plate 272 positioned over the curved surface 262, capturing the distal tab 296 of the anvil buttress 290 therebetween, as shown in FIG. 16. The retainer 231 of the loading tool 200 is then placed over the anvil tip 248 such that the peg 235 of the retainer 231 engages the spherical distal region 265a of the opening 265 defined through the anvil tip 248 to retain the anvil buttress 290 and the anvil tip 248 on the loading tool 200. In the loaded configuration, the proximal end portion 292a of the anvil buttress 290 is engaged with the proximal protrusion 232 extending from the support surface 224 of the carrier 220, and the distal end portion 292b of the anvil buttress 290 is captured within the anvil tip 248 by the retention assembly 270.

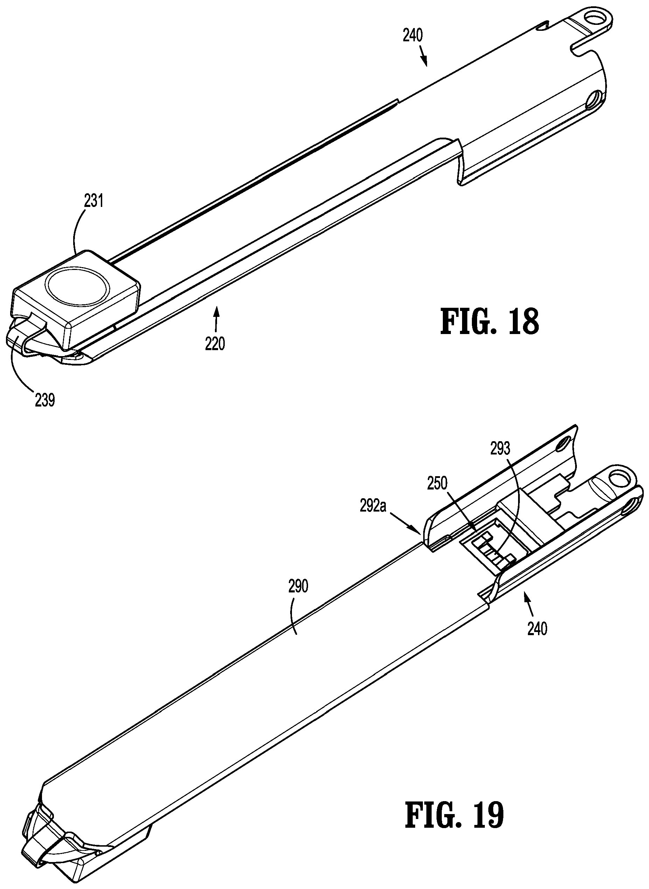

In a method of loading the anvil assembly 240 with the anvil buttress 290 and the anvil tip 248, the anvil plate 242 and the anvil cover 244 are slid distally into the cavity 221 of the carrier 220 such that the tissue facing surface 246 of the anvil plate 242 is adjacent to or in contact with the anvil buttress 290. The anvil assembly 240 is guided through and retained within the cavity 221 of the carrier 220 by the support surface 224, the longitudinal rails 226, and the flanges 228. The anvil assembly 240 is slid distally until it is fully advanced with the carrier 220, as shown in FIGS. 18 and 19, and the catch 268 of the beam 266 engages a groove 242c defined in the anvil plate 242, as seen in FIG. 22, locking the anvil tip 248 to the anvil plate 242. As seen in FIG. 19, the proximal window 293 of the anvil buttress 290 is aligned with the hook assembly 250 of the anvil plate 242 when fully inserted into the loading tool 200 such that the hooks 252 extend through the proximal window 293 and retain the proximal end portion 292a of the anvil buttress 290 to the anvil assembly 240.

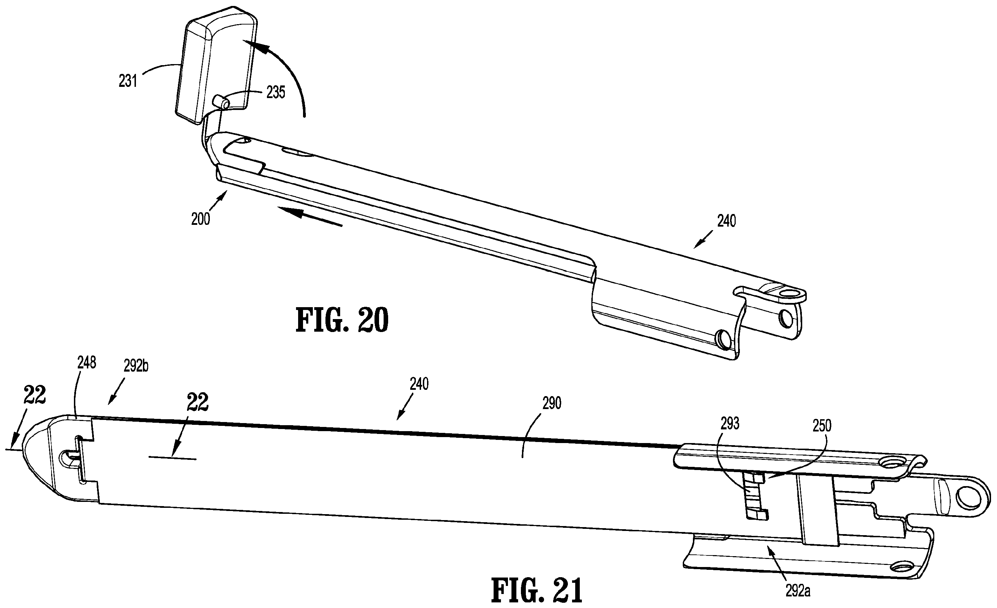

The loading tool 200 is separated from the anvil assembly 240 by removing (e.g., flipping) the retainer 231 out of the anvil tip 248, as shown in FIG. 20, and sliding the loading tool 200 and/or the anvil assembly 240 away from each other. As shown in FIG. 21, the proximal end portion 292a of the anvil buttress 290 is retained on the anvil assembly 240 by engagement of the hook assembly 250 through the proximal window 293 and the distal end portion 292b of the anvil buttress 290 is retained on the anvil assembly 240 by the anvil tip 248. Specifically, the distal end portion 292b of the anvil buttress 290 is retained on the anvil assembly 240 by engagement of the retention assembly 270 with the distal tab 296 as described above.

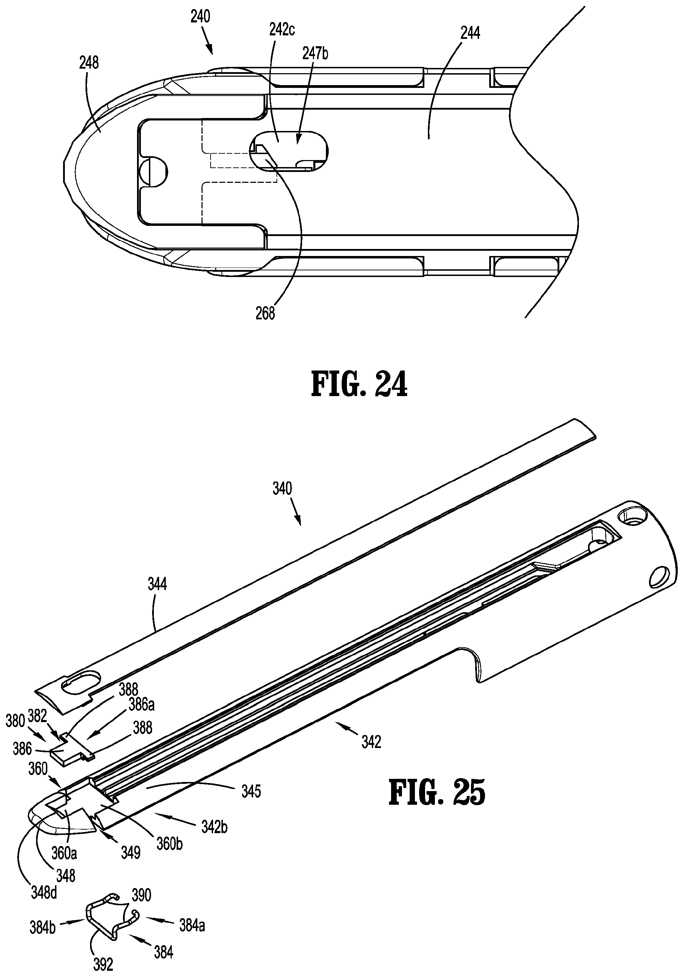

The surgical stapling apparatus 1 (FIG. 1), with the anvil assembly 240 loaded with the anvil buttress 290, is ready for use. In use, as shown in FIG. 23, at the end of a firing stroke, the I-beam 84 cams the retention assembly 270 distally, aligning the slot 273 of the plate 272 with the slotted proximal region 265b of the anvil tip 248 and releasing the distal tab 296 of the anvil buttress 290. After use, the peg 244 (FIG. 20) on the loading tool 200 may be utilized through the opening 247b defined through the cover plate 244, as seen in FIG. 24, to move the catch 268 out of the groove 242c of the anvil plate 242 to release the anvil tip 248 from the anvil assembly 240.

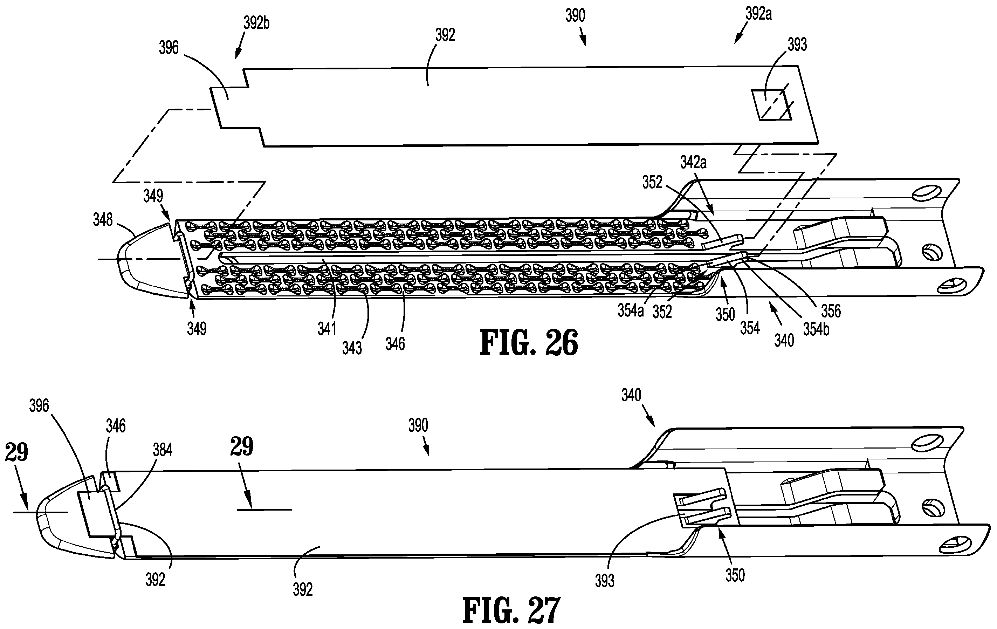

Turning now to FIGS. 25 and 26, an anvil assembly 340 in accordance with yet another aspect of the present disclosure is shown. The anvil assembly 340 includes an anvil plate 342 and a cover plate 344 secured over the anvil plate 342. The anvil plate 342 has a central longitudinal slot 341 formed therein and a plurality of staple forming pockets or cavities 343 defined in an inward or tissue facing surface thereof 346, with an anvil tip 348 extending distal to the staple forming pockets 343. A pair of recesses 349 are defined in side edges of the anvil assembly 340 between the anvil plate 342 and the anvil tip 348. A proximal portion 342a of the anvil plate 342, which extends proximally of the staple forming pockets 343, includes a hook assembly 350 configured to secure a proximal end portion of a surgical buttress thereto. The anvil tip 348 includes a spring assembly 380 configured to secure a distal end portion of the surgical buttress thereto.

The hook assembly 350 includes a pair of hooks 352, with each hook 352 disposed on opposed sides of the central longitudinal slot 341. Each hook 352 includes an arm 354 having a first or distal end portion 354a anchored or secured to the tissue facing surface 346 of the anvil plate 342 and a second or proximal end portion 354b including a finger 356 extending therefrom. The arm 354 extends upwardly away from the tissue facing surface 346 such that the finger 356 extends proximally and in spaced relation relative to the tissue facing surface 346 of the anvil assembly 340.

A cavity 360 is defined within the anvil tip 348 and the distal end portion 342b of the anvil plate 342. The cavity 360 is open to an outer surface 345 of the anvil assembly 340 at the anvil tip 348 and covered by the cover plate 344 at the distal end portion 342b of the anvil plate 342. A dimension or width of a distal portion 360a of the cavity 360 defined in the anvil tip 348 is smaller than a dimension or width of a proximal portion 360b of the cavity 360 defined in the anvil plate 342. The anvil tip 348 includes a sloped surface 348d extending from the outer surface 345 into the cavity 360.

The spring assembly 380 includes a slider 382 and a spring 384. The slider 382 includes a body 386 and a pair of wings 388 extending laterally from a proximal portion 386a of the body 386. The spring 384 has a rigid structure (e.g., a wire frame) having a proximal end portion 384a including a pair of legs 390 configured to extend around the proximal portion 386a of the body 386 of the slider 382 and a distal end portion 384b including a bridge 392 interconnecting the pair of legs 390.

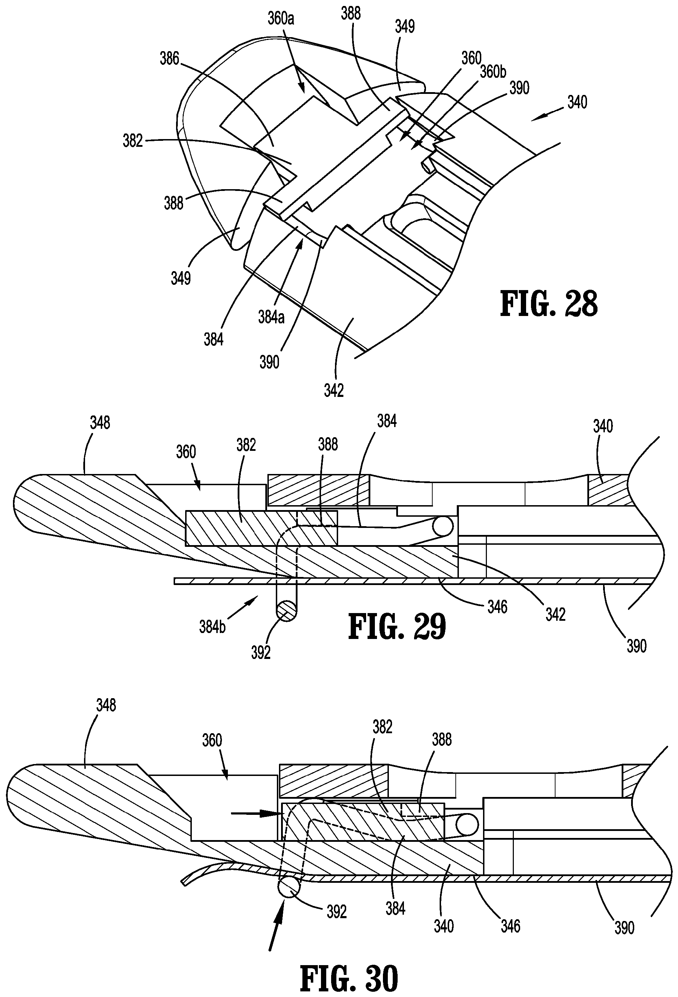

As shown in FIG. 28, the spring 384 is positioned in the cavity 360 of the anvil assembly 340 with the proximal end portion 384a disposed within proximal portion 360b of the cavity 360. Portions of the pair of legs 390 extend through the pair of recesses 349 defined in the anvil plate 342 such that the distal end portion 384b of the spring 384 is disposed outside of the cavity 360 and across the tissue facing surface 346 of the anvil plate 342 (FIG. 29). The slider 382 is positioned within the cavity 360 with the pair of wings 388 positioned over the pair of legs 390 of the spring 384 within the proximal portion 360b of the cavity 360 and the body 386 extending distally into the distal portion 360a of the cavity 360.

The spring assembly 380 has an unclamped or unload position, as shown in FIG. 29, in which the slider 382 is disposed in a distal position and the pair of wings 388 of the slider 382 cams the spring 384 distally such that the bridge 392 extends away from and in spaced relation relative to the tissue facing surface 346 of the anvil assembly 340. The spring assembly 380 also has a clamped or loaded position, as shown in FIG. 30, in which the slider 382 is disposed in a proximal position and the pair of wings 388 cams the spring 384 upwardly within the cavity 360 to move the bridge 392 into contact with the tissue facing surface 346 of the anvil assembly 340.

As shown in FIG. 27, the anvil buttress 390 includes a body 392 having a proximal end portion 392a including a proximal window 393 defined therethrough and a distal end portion 392b including a distal tab 396 extending therefrom. The distal tab 396 has a smaller dimension or width than the body 392.

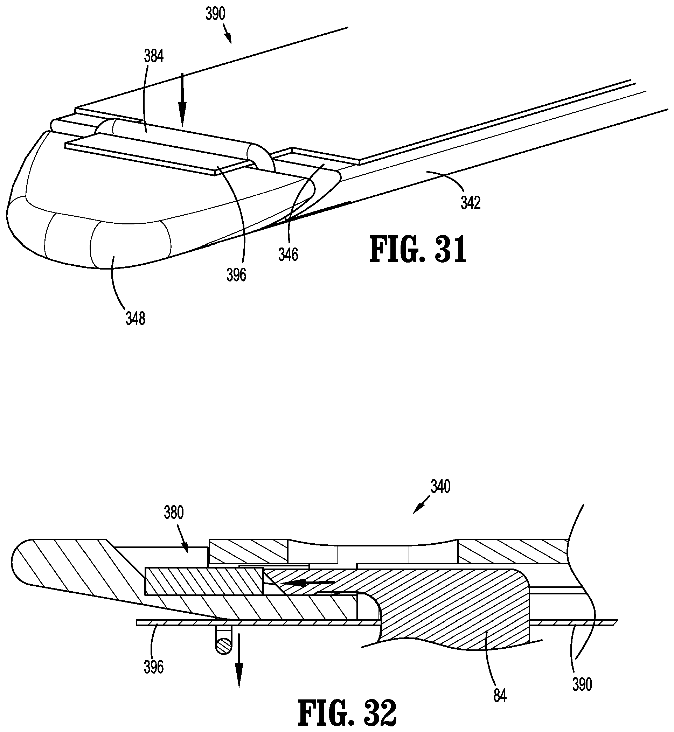

In a method of loading the anvil assembly 340 with the anvil buttress 390, the spring assembly 380 is positioned in the unload position as shown in FIGS. 26, 27, and 29. The body 392 of the anvil buttress 390 is placed against the tissue facing surface 346 of the anvil assembly 340 such that the proximal window 393 engages the hook assembly 350 and the distal tab 396 is positioned between the tissue facing surface 246 and the bridge 392 of the spring 384. The slider 382 is then moved to the load position, as seen in FIGS. 30 and 31, by accessing the slider 382 through the open distal portion 360a of the cavity 360 and pushing the slider 382 proximally, for example, with a loading tool (not shown).

The surgical stapling apparatus 1 (FIG. 1), with the anvil assembly 340 loaded with the anvil buttress 390, is ready for use. In use, as shown in FIG. 32, at the end of a firing stroke, the I-beam 84 cams the spring assembly 380 distally to the unload position, freeing the distal tab 396 of the anvil buttress 390 from the anvil assembly 340. The spring assembly 380 remains in the unload position until a new anvil buttress 390 is loaded onto the anvil assembly 340, as described above.

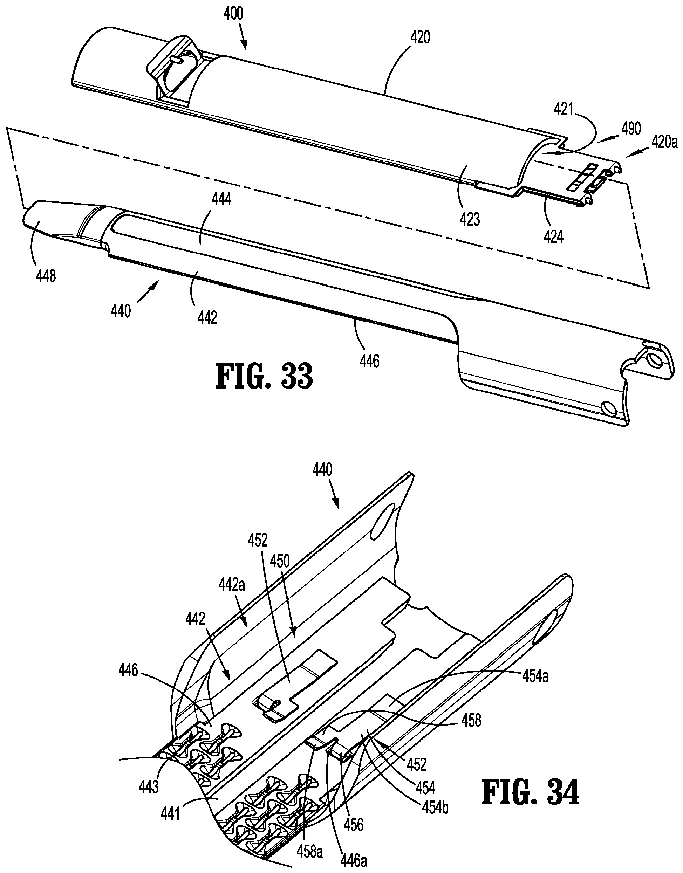

Referring now to FIG. 33, an anvil assembly 440 and an anvil buttress loading tool 400, in accordance with another aspect of the present disclosure, is shown. The anvil assembly 440 includes an anvil plate 442 and a cover plate 444 secured over the anvil plate 442. As shown in FIG. 34, in conjunction with FIG. 33, the anvil plate 442 has a central longitudinal slot 441 formed therein and a plurality of staple forming pockets or cavities 443 defined in an inward or tissue facing surface thereof 446, with an anvil tip 448 extending distal to the staple forming pockets 443. A proximal portion 442a of the anvil plate 442, which extends proximally of the staple forming pockets 443, includes a spring and hook assembly 450 configured to secure a proximal end portion of a surgical buttress thereto.

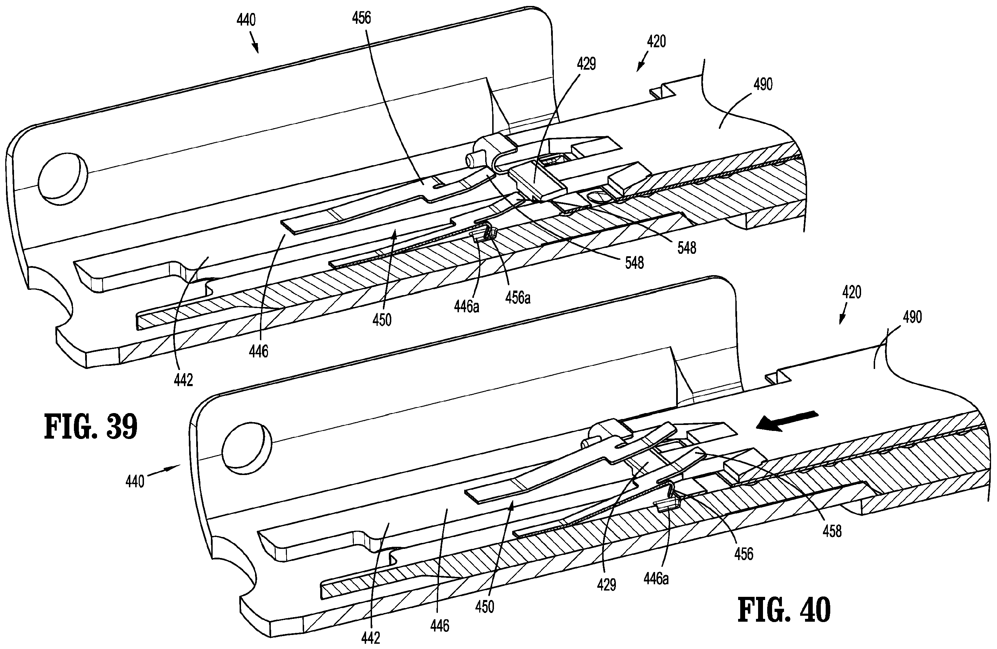

The spring and hook assembly 450 includes a pair of spring and hook members 452, with each spring and hook members 452 disposed on opposed sides of the central longitudinal slot 441. Each spring and hook member 452 includes an arm 454 having a first or proximal end portion 454a anchored or secured to the tissue facing surface 446 of the anvil plate 442 and a second or distal end portion 454b including first and second fingers 456, 458 extending therefrom. The first finger 456 is bent or curved relative to the arm 454 downwardly towards the tissue facing surface 446 such that a terminal end 456a (FIG. 39) of the first finger 456 is biased into a notch 446a defined in the tissue facing surface 446. The second finger 458 is disposed laterally inwardly of the first finger 456 and is bent or curved upwardly away from the tissue facing surface 446 such that a terminal end 458a of the second finger 458 is biased in spaced relation relative to the tissue facing surface 446. Each arm 454 is hingedly or springedly connected to the anvil plate 452 at the proximal end portion 454a thereof such that when a force acts upon the second fingers 458, the second finger 458 is lifted off of the tissue facing surface 446 and the first finger 456 is moved out of the notch 446a.

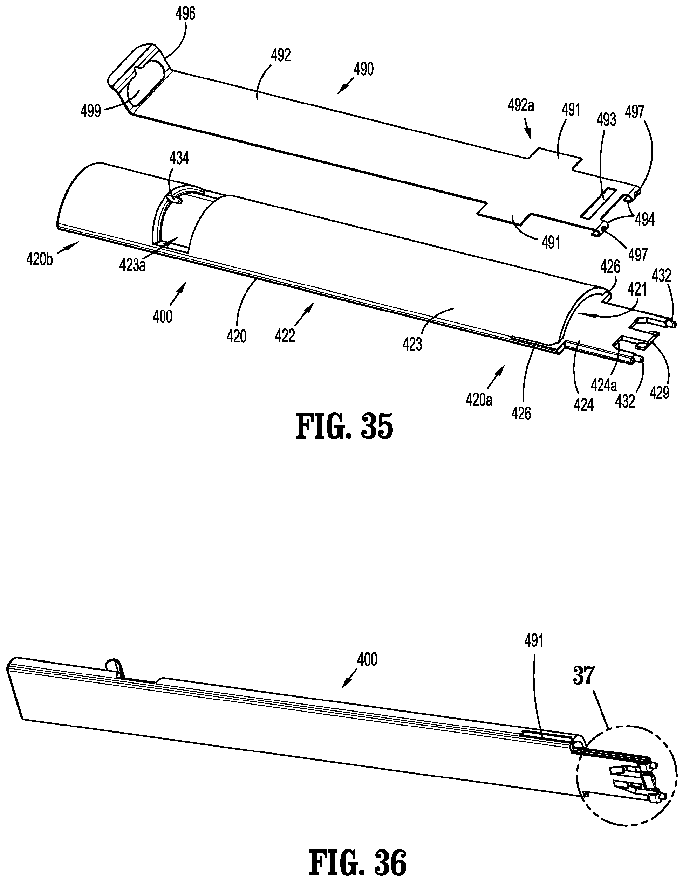

As shown in FIG. 35, the anvil buttress 490 includes a body 492 having a pair of wings 491 extending laterally from a proximal end portion 492a thereof. A first or proximal window 493 is defined through the proximal end portion 492a of the body 492 proximal of the pair of wings 491. A pair of proximal tabs 494 extend proximally from the proximal end portion 492a of the body 492, with each proximal tab 494 defining an opening 497 therethrough. A distal tab 496 extends distally from the distal end portion 492b of the body 492 and defines an opening 499 therethrough.

As shown in FIGS. 35-37, the anvil buttress loading tool 400 includes a carrier 420 is configured to releasably retain the anvil buttress 490 therein and to receive the anvil assembly 440 (FIG. 33) for loading of the anvil buttress 490 thereon. The carrier 420 includes a housing 422 having a support surface 424 and a cover 423 extending over the support surface 424. A cavity 421 is defined in the carrier 420 by the support surface 424 and the cover 423. A pair of slits 426 is defined between the support surface 424 and the cover 423 at a proximal end portion 420a of the carrier 420. The support surface 424 extends proximally beyond the cover 423 and includes a pair of proximal protrusions 432 extending proximally from a proximal end 424a of the support surface 424. A ramp 429 is disposed between the pair of proximal protrusions 342. The cover 423 includes an opening 423a defined therethrough at a distal end portion 420b of the carrier 420 and includes a distal protrusion 434 extending proximally into the opening 423a.

In a method of loading the loading tool 400 with the anvil buttress 490, the body 492 of the anvil buttress 490 is passed into the cavity 421 of the carrier 420 and positioned against the support surface 424 of the carrier 420 with the pair of wings 491 received within the pair of slits 426. With the body 492 of the anvil buttress 490 positioned on the support surface 424, the pair of proximal tabs 494 is aligned or in registration with the pair of proximal protrusions 432 of the support surface 424, and the distal tab 496 is aligned or in registration with the distal protrusion 434 of the cover 423. Accordingly, the proximal and distal end portions 492a, 492b of the anvil buttress 490 are manipulated to engage the pair of proximal tabs 494 with the pair of proximal protrusions 432 and the distal tab 496 through the opening 423a and into engagement with the distal protrusion 434.

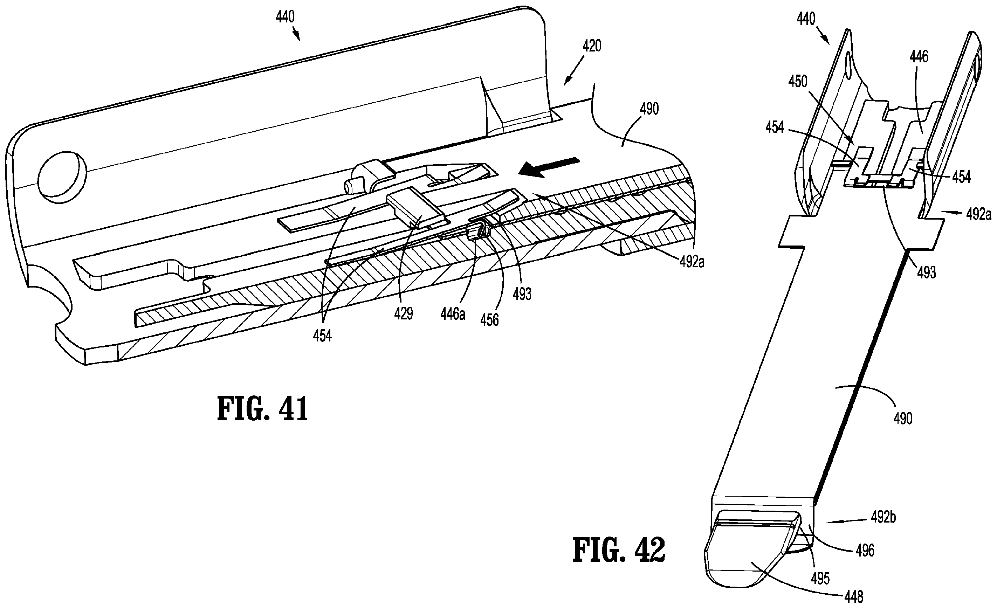

In a method of loading the anvil assembly 440 with the anvil buttress 490, as seen initially in FIG. 33, the anvil tip 448 of the anvil assembly 440 is aligned a proximal end 420a of the carrier 420 of the loading tool 400. The anvil assembly 440 is slid distally into the cavity 421 of the carrier 420 such that the tissue facing surface 446 of the anvil assembly 440 is adjacent to or in contact with the anvil buttress 490. The anvil assembly 440 is guided through and retained within the cavity 421 of the carrier 420 by the support surface 424 and the cover 423. The anvil assembly 440 is slid distally until the anvil tip 448 extends through the distal window 495 defined through the anvil buttress 490. During this sliding movement, the ramp 429 of the carrier 420 contacts the second fingers 548 of the spring and hook assembly 450, raising the second fingers 548 off of the tissue facing surface 446 of the anvil plate 442 and lifting the first fingers 456 out of the notches 446a defined in the tissue facing surface 446, as shown in FIGS. 39 and 40. Once the ramp 429 clears the second fingers 458, as shown in FIG. 41, the arms 454 snaps back down and the first fingers 456 extend through the proximal window 493 and re-enter the notches 446a thereby capturing the proximal end portion 492a of the anvil buttress 490 to the anvil assembly 440.

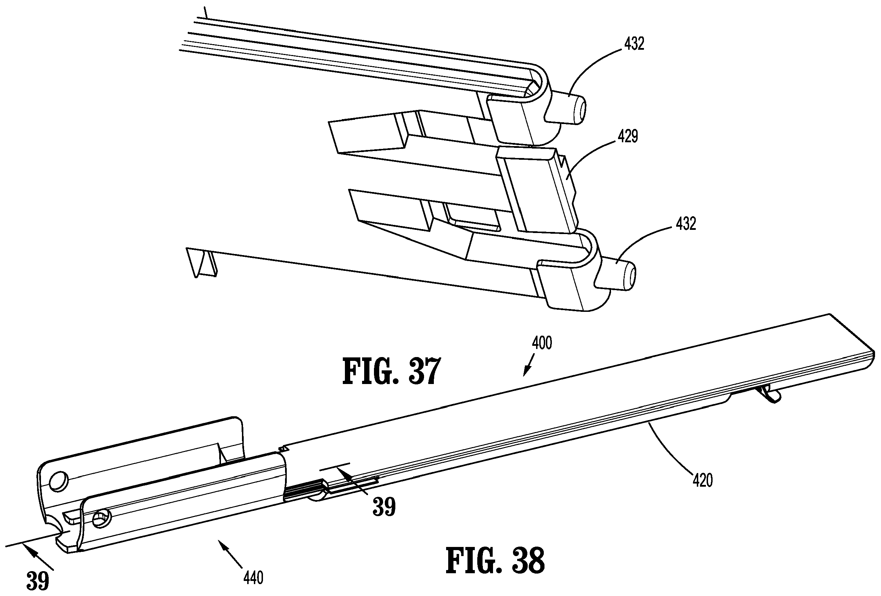

Once the anvil assembly 440 is fully advanced into the carrier 420 of the loading tool 400, as seen in FIG. 38, the loading tool 400 may be separated from the anvil assembly 440 by sliding the loading tool 400 and/or the anvil assembly 440 in opposite directions. As the anvil assembly 440 and the loading tool 400 are separated from each other, the arms 454 of the spring and hook assembly 450 elastically deform downwardly into the tissue facing surface 446 of the anvil assembly 440 until the ramp 429 passes thereover. The proximal end portion 492a of the anvil buttress 490 is retained on the anvil assembly 440 by engagement of the spring and hook assembly 450 through the proximal window 493 and the distal end portion 492b of the anvil buttress 490 is retained on the anvil assembly 440 by engagement of the distal tab 496 with the anvil tip 448 through the distal window 495, as seen in FIG. 42. The surgical stapling apparatus 1 (FIG. 1), with the anvil assembly 440 loaded with the anvil buttress 490, is ready for use.

## Claims

1. An anvil buttress loading assembly comprising:
an anvil buttress loading tool (100, 200, 400) including a carrier (120, 220, 420) defining a cavity (121, 221, 421) therein, the carrier having a support surface (124, 224, 424), longitudinal rails (126, 226) extending from opposed sides of the support surface, a plurality of axially spaced and opposed tabs (128) extending from the longitudinal rails and over the support surface, a proximal end portion (120a, 420a) of the carrier including a pair of proximal protrusions (132, 232, 432) and a distal end portion (220b, 420b) of the carrier including a distal protrusion (132, 134, 434); and
an anvil buttress (90, 290, 390, 490) including a body (92, 292, 392, 492), a pair of proximal tabs (94, 294, 494) extending proximally from the body, and a distal tab (96, 296, 396, 496) extending distally from the body,
wherein the body of the anvil buttress is positioned on the support surface, the pair of proximal tabs engaged with the pair of proximal protrusions, and wherein the distal tab is engaged with the distal protrusion.

2. The anvil buttress loading assembly according to claim 1, wherein the carrier of the anvil buttress loading tool includes a transverse rail (130) extending across distal ends (126a) of the longitudinal rails and over the support surface defining a distal opening (125) into the cavity.

3. The anvil buttress loading assembly according to claim 2, wherein the distal protrusion extends from the transverse rail.

4. The anvil buttress loading assembly according to any one of claims 1 to 3, wherein the anvil buttress includes a distal window (95, 495) defined through a distal end portion (92b, 292b, 392b, 492b) of the body, the distal window aligned with the distal opening of the carrier.

5. An anvil buttress loading system comprising:
an anvil assembly (40, 240, 340, 440) including an anvil plate (42, 242, 342 442) and an anvil tip (48 248, 348, 448), the anvil plate having a tissue facing surface (46, 246, 346, 446) defining a plurality of staple forming pockets (43, 243, 343, 443) therein, a proximal end portion of the tissue facing surface including a hook assembly (50, 250, 350, 450);
an anvil buttress loading tool (100, 200, 400) and anvil buttress (90, 290, 390, 490) according to any one of the preceding claims, wherein:
the anvil assembly is positionable between the longitudinal rails of the anvil buttress loading tool with the tissue facing surface adjacent to the support surface, and the body of the anvil buttress is positionable on the tissue facing surface of the anvil assembly with a proximal end portion of the body engaged with the hook assembly and a distal end portion engaged with the anvil tip to retain the anvil buttress on the anvil assembly;
and the anvil buttress is transferrable from the anvil buttress loading tool to the anvil assembly.

6. The anvil buttress loading system according to claim 5, wherein the hook assembly of the anvil assembly includes a pair of hooks (52, 252, 352) disposed on opposed sides of a central longitudinal slot (41, 241, 341, 441) defined through the tissue facing surface, each hook including an arm (54, 254, 354, 454) having a first end anchored to the tissue facing surface and a second end including a finger (56, 256, 356, 456) extending therefrom.

7. The anvil buttress loading system according to claim 6, wherein the first end of the arm is anchored to the tissue facing surface at a proximal end thereof and the second end is disposed distal thereto.

8. The anvil buttress loading system according to claim 6 or claim 7, wherein a terminal end of the finger is biased against the tissue facing surface of the anvil assembly and movable relative to the tissue facing surface.

9. The anvil buttress loading system according to claim 6, claim 7 or claim 8, wherein the anvil buttress has a proximal window (93, 293, 393, 493) defined in the proximal end portion of the body, the proximal window configured to receive the fingers of the pair of hooks therethrough.

## Patentansprüche

1. Ambossversteifungsladeanordnung, umfassend:
ein Ambossversteifungsladewerkzeug (100, 200, 400), das einen Träger (120, 220, 420) beinhaltet, der in seinem Inneren einen Hohlraum (121, 221, 421) definiert, wobei der Träger eine Stützfläche (124, 224, 424), Längsschienen (126, 226), die sich von gegenüberliegenden Seiten der Stützfläche aus erstrecken, eine Vielzahl von axial beabstandeten und einander gegenüberliegende Laschen (128), die sich von den Längsschienen und über die Stützfläche erstrecken, einen proximalen Endabschnitt (120a, 420a) des Trägers, der ein Paar proximaler Vorsprünge (132, 232, 432) beinhaltet, und einen distalen Endabschnitt (220b, 420b) des Trägers aufweist, der einen distalen Vorsprung (132, 134, 434) beinhaltet; und
eine Ambossversteifung (90, 290, 390, 490), die einen Körper (92, 292, 392, 492), ein Paar proximaler Laschen (94, 294, 494), die sich proximal von dem Körper aus erstrecken, und eine distale Lasche (96, 296, 396, 496) beinhaltet, die sich distal von dem Körper aus erstreckt, wobei der Körper der Ambossversteifung auf der Stützfläche positioniert ist, wobei das Paar proximaler Laschen mit dem Paar proximaler Vorsprünge in einem Eingriff steht und wobei die distale Lasche mit dem distalen Vorsprung in einem Eingriff steht.

2. Ambossversteifungsladeanordnung nach Anspruch 1, wobei der Träger des Ambossversteifungsladewerkzeugs eine Querschiene (130) beinhaltet, die sich über distale Enden (126a) der Längsschienen und über die Stützfläche erstreckt und die eine distale Öffnung (125) in den Hohlraum definiert.

3. Ambossversteifungsladeanordnung nach Anspruch 2, wobei sich der distale Vorsprung von der Querschiene aus erstreckt.

4. Ambossversteifungsladeanordnung nach einem der Ansprüche 1 bis 3, wobei die Ambossversteifung ein distales Fenster (95, 495) beinhaltet, das durch einen distalen Endabschnitt (92b, 292b, 392b, 492b) des Körpers definiert wird, wobei das distale Fenster auf die distale Öffnung des Trägers ausgerichtet ist.

5. Ambossversteifungsladesystem, umfassend:
eine Ambossanordnung (40, 240, 340, 440), die eine Ambossplatte (42, 242, 342 442) und eine Ambossspitze (48 248, 348, 448) beinhaltet, wobei die Ambossplatte eine dem Gewebe zugewandte Oberfläche (46, 246, 346, 446) aufweist, die in ihrem Inneren eine Vielzahl von stapelbildenden Taschen (43, 243, 343, 443) definiert, wobei ein proximaler Endabschnitt der dem Gewebe zugewandten Oberfläche eine Hakenanordnung (50, 250, 350, 450) beinhaltet;
ein Ambossversteifungsladewerkzeug (100, 200, 400) und eine Ambossversteifung 90, 290, 390, 490) nach einem der vorhergehenden Ansprüche, wobei:
die Ambossanordnung zwischen den Längsschienen des Ambossversteifungsladewerkzeugs positionierbar ist, wobei die dem Gewebe zugewandte Oberfläche benachbart zur Stützfläche ist, und wobei der Körper der Ambossversteifung auf der dem Gewebe zugewandten Oberfläche der Ambossanordnung positionierbar ist, wobei ein proximaler Endabschnitt des Körpers mit der Hakenanordnung in einem Eingriff steht und wobei ein distaler Endabschnitt mit der Ambossspitze in einem Eingriff steht, um die Ambossversteifung an der Ambossanordnung zu halten;
und wobei die Ambossversteifung von dem Ambossversteifungsladewerkzeug zu der Ambossanordnung transferierbar ist.

6. Ambossversteifungsladesystem nach Anspruch 5, wobei die Hakenanordnung der Ambossanordnung ein Paar Haken (52, 252, 352) beinhaltet, die auf gegenüberliegenden Seiten eines zentralen Längsschlitzes (41, 241, 341, 441) angeordnet sind, der durch die dem Gewebe zugewandten Oberfläche definiert wird, wobei jeder Haken einen Arm (54, 254, 354, 454) beinhaltet, der ein erstes Ende, das an der dem Gewebe zugewandten Oberfläche verankert ist, und ein zweites Ende aufweist, das einen Finger (56, 256, 356, 456) beinhaltet, der sich davon erstreckt.

7. Ambossversteifungsladesystem nach Anspruch 6, wobei das erste Ende des Arms an einem proximalen Ende der dem Gewebe zugewandten Oberfläche verankert ist und wobei das zweite Ende distal dazu angeordnet ist.

8. Ambossversteifungsladesystem nach Anspruch 6 oder Anspruch 7, wobei ein Endstück des Fingers gegen die dem Gewebe zugewandte Oberfläche der Ambossanordnung vorgespannt und relativ zu der dem Gewebe zugewandten Oberfläche beweglich ist.

9. Ambossversteifungsladesystem nach Anspruch 6, Anspruch 7 oder Anspruch 8, wobei die Ambossversteifung ein proximales Fenster (93, 293, 393, 493) aufweist, das in dem proximalen Endabschnitt des Körpers definiert ist, wobei das proximale Fenster konfiguriert ist, um die Finger des Hakenpaars durch das Fenster aufzunehmen.

## Revendications

1. Ensemble de chargement de contrefort d'enclume, comprenant :
un outil de chargement de contrefort d'enclume (100, 200, 400) incluant un élément porteur (120, 220, 420) définissant une cavité (121, 221, 421) dans celui-ci, l'élément porteur ayant une surface de support (124, 224, 424), des rails longitudinaux (126, 226) s'étendant depuis des côtés opposés de la surface de support, une pluralité de pattes axialement espacées et opposées (128) s'étendant depuis les rails longitudinaux et par-dessus la surface de support, une partie d'extrémité proximale (120a, 420a) de l'élément porteur incluant une paire de saillies proximales (132, 232, 432) et une partie d'extrémité distale (220b, 420b) de l'élément porteur incluant une saillie distale (132, 134, 434) ; et
un contrefort d'enclume (90, 290, 390, 490) incluant un corps (92, 292, 392, 492), une paire de pattes proximales (94, 294, 494) s'étendant proximalement depuis le corps, et une patte distale (96, 296, 396, 496) s'étendant distalement depuis le corps,
dans lequel le corps du contrefort d'enclume est positionné sur la surface de support, la paire de pattes proximales étant en prise avec la paire de saillies proximales, et dans lequel la patte distale est en prise avec la saillie distale.

2. Ensemble de chargement de contrefort d'enclume selon la revendication 1, dans lequel l'élément porteur de l'outil de chargement de contrefort d'enclume inclut un rail transversal (130) s'étendant d'un côté à l'autre d'extrémités distales (126a) des rails longitudinaux et par-dessus la surface de support définissant une ouverture distale (125) dans la cavité.

3. Ensemble de chargement de contrefort d'enclume selon la revendication 2, dans lequel la saillie distale s'étend depuis le rail transversal.

4. Ensemble de chargement de contrefort d'enclume selon l'une quelconque des revendications 1 à 3, dans lequel le contrefort d'enclume inclut une fenêtre distale (95, 495) définie à travers une partie d'extrémité distale (92b, 292b, 392b, 492b) du corps, la fenêtre distale étant alignée avec l'ouverture distale de l'élément porteur.

5. Système de chargement de contrefort d'enclume, comprenant :
un ensemble d'enclume (40, 240, 340, 440) incluant une plaque d'enclume (42, 242, 342 442) et un bout d'enclume (48 248, 348, 448), la plaque d'enclume ayant une surface faisant face à tissu (46, 246, 346, 446) définissant une pluralité de poches de formation d'agrafe (43, 243, 343, 443) dans celle-ci, une partie d'extrémité proximale de la surface faisant face à tissu incluant un ensemble de crochet (50, 250, 350, 450) ;
un outil de chargement de contrefort d'enclume (100, 200, 400) et un contrefort d'enclume (90, 290, 390, 490) selon l'une quelconque des revendications précédentes, dans lequel :
l'ensemble d'enclume est positionnable entre les rails longitudinaux de l'outil de chargement de contrefort d'enclume avec la surface faisant face à tissu adjacente à la surface de support, et le corps du contrefort d'enclume est positionnable sur la surface faisant face à tissu de l'ensemble d'enclume avec une partie d'extrémité proximale du corps en prise avec l'ensemble de crochet et une partie d'extrémité distale en prise avec le bout d'enclume pour retenir le contrefort d'enclume sur l'ensemble d'enclume ;
et le contrefort d'enclume est transférable depuis l'outil de chargement de contrefort d'enclume à l'ensemble d'enclume.

6. Système de chargement de contrefort d'enclume selon la revendication 5, dans lequel l'ensemble de crochet de l'ensemble d'enclume inclut une paire de crochets (52, 252, 352) disposés sur des côtés opposés d'une fente longitudinale centrale (41, 241, 341, 441) définie à travers la surface faisant face à tissu, chaque crochet incluant un bras (54, 254, 354, 454) ayant une première extrémité ancrée à la surface faisant face à tissu et une seconde extrémité incluant un doigt (56, 256, 356, 456) s'étendant depuis celle-ci.

7. Système de chargement de contrefort d'enclume selon la revendication 6, dans lequel la première extrémité du bras est ancrée à la surface faisant face à tissu au niveau d'une extrémité proximale de celle-ci et la seconde extrémité est disposée distalement à celle-ci.

8. Système de chargement de contrefort d'enclume selon la revendication 6 ou la revendication 7, dans lequel une extrémité terminale du doigt est sollicitée contre la surface faisant face à tissu de l'ensemble d'enclume et mobile relativement à la surface faisant face à tissu.

9. Système de chargement de contrefort d'enclume selon la revendication 6, la revendication 7, ou la revendication 8, dans lequel le contrefort d'enclume a une fenêtre proximale (93, 293, 393, 493) définie dans la partie d'extrémité proximale du corps, la fenêtre proximale étant configurée pour recevoir le doigts de la paire de crochets à travers celle-ci.
